# EUROPEAN PATENT SPECIFICATION

(11) **EP 1 125 134 B1**
(45) Date of publication and mention of the grant of the patent: **04.11.2009**
(21) Application number: 99960224.6
(22) Date of filing: 05.11.1999
(51) Int. Cl.: G01N 33/68, C07K 14/47

(54) **S100 PROTEINS AND AUTOANTIBODIES AS SERUM MARKERS FOR CANCER**
S100-PROTEINE UND -AUTOANTIKÖRPER ALS SERUMMARKER FÜR KREBS
PROTEINES S100 ET AUTO-ANTICORPS COMME MARQUEURS SERIQUES POUR LE CANCER

(30) Priority: 05.11.1998 US 107209 P
(43) Date of publication of application: 22.08.2001
(73) Proprietor: THE REGENTS OF THE UNIVERSITY OF MICHIGAN, Ann Arbor, MI 48109-0510 (US)
(72) Inventor: HANASH, Samir, M., Ann Arbor, MI 48105 (US); MISEK, David, Ann Arbor, MI 48108 (US); PRASANAN, Latha, Ann Arbor, MI 48103 (US)
(74) Representative: Lucas, Brian Ronald
(86) International application number: PCT/US1999/026226
(87) International publication number: WO 2000/026668

(56) References cited:
- EP-A- 0 585 201
- WO-A-92/18535
- WO-A-98/01471
- WO-A-98/35985
- WO-A-98/37420
- RU-A- 2 107 913
- US-A- 5 350 687
- US-A- 5 405 749
- DATABASE WPI Week 9624 Derwent Publications Ltd., London, GB; AN 1996-238053 XP002136800 "Screening of psyco-nervous illnesses - using diagnostic ELITEST-1 system for cheaper and more rapid work" & RU 2 045 759 C (RUBTSOVA M YU I), 10 October 1995 (1995-10-10)
- MELANOMA RESEARCH, vol. 7, no. 5, 1997, pages 393-399, XP000883609
- MELANOMA RESEARCH, vol. 6, no. 2, 1996, pages 133-137, XP000886310
- ANTICANCER RESEARCH, vol. 17, no. B4, 1997, pages 3071-3073, XP000886285
- CELIS J.E. ET AL: 'Bladder squamous cell carcinomas express psoriasin and externalize it to the urine' THE JOURNAL OF UROLOGY vol. 155, 1996, USA, pages 2105 - 2112, XP005576162
- WATSON P.H. ET AL: 'Molecules in focus: Psoriasin (S100A7)' THE INTERNATIONAL JOURNAL OF BIOCHEMISTRY AND CELL BIOLOGY vol. 30, 1998, pages 567 - 571, XP002971433
- TANAKA ET AL: 'Human calgizzarin; one colorectal cancer-related gene selected by a large scale random cDNA sequencing and Northern blot analysis' CANCER LETTERS vol. 89, 1995, IRELAND, pages 195 - 200
- MOOG-LUTZ; BOUILLET; REGNIER ET AL: 'Comparative expression of the Psoriasin and S100C genes in breast carcinoma and co-localization to human chromosome 1q21-q22' INT. J. CANCER vol. 63, 1995, pages 297 - 303, XP008065725
- TOMASETTO; REGNIER; MOOG-LUTZ ET AL: 'Identification of four novel human genes amplified and overexpressed in breast carcinoma and localized to the q11 - q21.3 region of chromosome 17' GENOMICS vol. 28, 1995, pages 367 - 376

## Description

### 1. INTRODUCTION

The present invention relates to screening methods for diagnosis, prognosis or susceptibility to cancer in a subject by means of detecting the presence of serum autoantibodies to specific 5100 protein antigens. The method of the invention can also be used to identify subjects at risk for developing cancer. The method of the invention involves the use of subject derived serum samples, or other biological fluid samples. Kits can be used to screen subjects for the detection of autoantibodies to S100 proteins, as a diagnostic, predictive or prognostic indicator of cancer. Elevated levels of circulating autoantibodies reactive against several S100 proteins were detected in the sera of cancer subjects.

### 2. BACKGROUND OF THE INVENTION

A number of cellular proteins have been demonstrated to occur at increased levels in body fluids of subjects with different types of cancer. The increased levels of such proteins in cancer subjects provide diagnostic and prognostic assays for the presence of cancer. For example, elevated serum levels of prostate specific antigen (PSA) is frequently used as an indicator of the presence of prostate cancer in subjects. The occurrence of antibodies to tumor derived proteins as in the case of mutant p53 has also been noted to occur in some cancers. However, there is little knowledge that allows prediction of which proteins in tumors may be antigenic or may occur at increased levels in body fluids.

S100 proteins are low-molecular-weight calcium binding proteins that are believed to play an important role in various Cellular processes such as cytoskeletal/membrane interaction, cell division and differentiation. The S100 family of proteins consists of at least 17 family members and the genes encoding this family of proteins have been localised to human chromosomes 1q21. The S100 proteins possess a characteristic EF-hand domain which binds calcium with high affinity. The designation S100 protein refers to the original protein; other S100 proteins are referred to specifically as, for example, S100-A7, S100-A8, etc.

Two specific S100 proteins, m S100-A8 and S100-A9, also referred to as myeloid related protein-8 (MRP-8) and myeloid related protein-14(MRP-14), are composed of two distinct EF-hands flanked by hydrophobic regions at either terminus separated by a central hinge region. S100-A8 has been demonstrated to mediate chemotactic activity on marcophages and a peptide encoded by the hinge region (between the two EF-hands) has been shown to specifically mediate this effect. S100-A8 and S100-A9 have both been shown to be secreted from granulocytes and monocytes, although it is presently unclear how these proteins are secreted as they do not possess a classical signal peptide. One possibility is that calcium binding may expose a hydrophobic domain which could allow an interaction with the membrane, thereby resulting in secretion of the molecules. Both S100-A8 and S100-A9 homodimerize and heterodimerize with each other, thus forming complexes of various molecular weights. An antibody against the cystic fibrosis antigen (an epitope formed by heterodimerization of S100-A8 and S100-A9) also will react positively against a 14kDa antigen which has been shown to be S100-A9.

WO98/35985 describes a diagnostic method for lung tumors based on the detection of at least one protein which is overexpressed in lung tumors. This document mentions MRP8 and MRP14.

The expression of S100 protein has been evaluated in a variety of disorders. For example, S100 proteins have been evaluated as serum markers for subjects with inflammatory diseases, including ulcerative colitis, Crohn's disease and as serum markers for subjects with infectious diseases including AIDs and malaria and for subjects with haematological disease. US 5,350,687 describes polypeptides MRP-8 and MRP-14, processes for their preparation, mRNAs, DNAs and hybrid vectors coding for said polpeptides, hosts transformed with such a vector, monoclonal and polyclonal antibodies to said polypeptides and diagnostic methods for inflammatory conditions and cystic fibrosis.

RU-C-2045759 describes the diagnosis of psycho-nervous illnesses by carrying out a quantitative estimation of autoantibodies to S100 protein.

EP 0 585 201 describes a diagnostic test system for detecting MRP-8 and MRP-14.

Evidence has accumulated that indicates that some S100 proteins can alter cellular invasion and metastatic spread of cancer. For example, S100 protein is expressed in dendritic cells in human transitional cell carcinoma of the bladder and the invasive potential of these tumars has been found to correlate with the presence of S100 protein expressing cells (Inoue et al., 1993, Virchows Arch A 422:351-355). Additionally, the expression of S100A4 correlates with the *in vitro* invasive potential of glioma cells (Merzak, et al., 1994, Neuropathol App.Neurobiol. 20-614-619).

Celis et al in J. Urology, 155, (1996), 2105 - 2112 suggests that psoriasin, a synonym name for S100-A7, is a potential marker for squamous cell carcinoma in humans. Watson et al in Int. J. Biochem & Cel Biology, 30, (1998), 567-571 also suggests that S100-A7 can play such a role and further suggests that it may have a potential role as a clinical marker of an important aspect of cancer pathology.

Expression of S100 proteins has also been evaluated as a serum marker for melanoma (Henze et al, 199, Dermatology 194:208-212; Buer et al, 1997, Brit.J.Cancer 75:1373-1376, Sherbet et al, 1998, Anticancer Research 18:2415-2422). However, S100 proteins have not been previously considered a serum markers for cancer in general, nor that their detectability in serum might have prognostic or therapeutic significance in cancer. Additionally, there have not been prior findings of autoantibodies to S100 proteins in cancer.

The invention relates to diagnostic evaluation and prognosis of cancer by detecting autoantibodies to S100 protein antigens in the serum of subjects with cancer or with precancerous lesions. The detection of increased serum levels of autoantibodies to S100 proteins constitutes a novel strategy for screening, diagnosis and prognosis of cancer.

The present invention relates to the use of the S100 protein antigens in immunoassays designed to detect the presence of serum autoantibodies to the S100 protein antigens. Such immunoassays can be utilized for diagnosis and prognosis of cancer. In accordance with the invention, measurement of S100 autoantibody levels in a subject's serum can be used for the early diagnosis of cancer. Moreover, the monitoring of serum autoantibody levels can be used prognostically to stage progression of the disease.

Pre-packaged diagnostic kits can be conveniently used in clinical settings, to diagnose patients having cancer or a predisposition to developing cancer. The kits can also be utilized to monitor the efficiency of compounds used for treatment of cancer.

An increase in the level of specific S100 proteins was detected in serum samples derived from subjects with lung cancer as well as colon cancer. Additionally, a number of S100 family members were shown to be secreted from breast cancer cells indicating that detection of these specific proteins in body fluid samples can be used for diagnosis and prognosis of subjects with breast cancer. In a specific embodiment, autoantibodies against specific S100 proteins were detected in the serum of subjects with cancer. The finding that levels of S100 proteins and S100 autoantibodies are increased in serum of cancer subjects provides a basis for development of diagnostic and prognostic methods as well as a means for monitoring the efficacy of various therapeutic treatments for cancer.

### 4. BRIEF DESCRIPTION OF THE DRAWINGS

Figure 1 A-B. Two dimensional electrophoretic gel patterns of normal colon (Figure 1A) and a colon tumor (Figure 1B) from the same subject. The location of some of the S100 proteins is identified with arrows. MRP-14 corresponds to S100-A9 and MRP-8 corresponds to S100-A8.
Figure 2. Two dimensional patterns of secreted proteins from breast cancer cells identifying the location of S100 proteins.

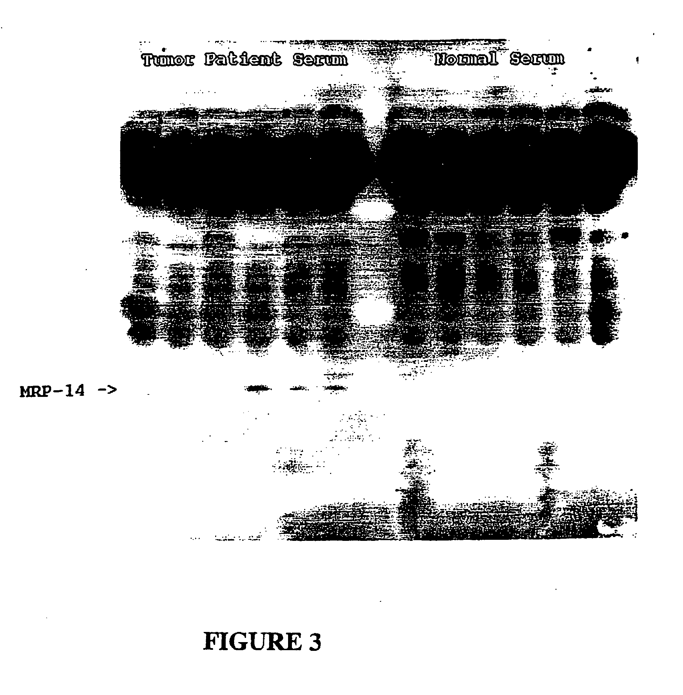

Figure 3. Detection of an protein band corresponding to S100-A9 in serum of lung cancer subjects.
Figure 4A. Western blot of a lung cancer treated with a serum from a subject with lung adenocarcinoma tumor.
Figure 4B. Western Blot of lung cancer treated with serum from a normal subject.

### 5. DETAILED DESCRIPTION OF THE INVENTION

The preheat invention achieves a highly desirable objective, namely providing methods for the diagnostic and prognostic evaluation of subjects with cancer and the identification of subjects exhibiting a predisposition to developing cancer. The assays of the invention comprise methods designed to detect the presence of S100 autoantibodies, in serum or other biological fluids of a subject.

The present invention encompasses a method for diagnosis and prognosis of a subject with lung cancer, comprising:
(a) contacting a biological fluid sample derived from a subject with a sample containing S100 protein antigens selected from the group consisting of S100-A9 and Calgizzarin under conditions such that a specific antigen-antibody binding can occur: and
(b) detecting the presence of immunospecific binding of autoantibodies to said protein in the subject's biological fluid sample,
wherein the presence of autoantibodies indicates the presence of lung cancer.

In a specific embodiment of invention, any member of the S100 protein family can be purified and utilized to screen a subject's serum for the presence of circulating autoantibodies to such protein antigens, by means of sensitive and rapid immunoadsorbent assays or by other procedures.

Kits for carrying out the above-described methods are described. The methods can be performed, for example, by utilizing pre-packaged diagnostic kits comprising an S100 peptide for detection of S100 autoantibodies in a subject derived sample.

The present invention is based on the discovery that levels of S100 proteins in serum are increased is subject with cancer such as lung cancer or colon cancer. In addition, breast cancer cells were demonstrated to secrete an S100 protein thus indicating that detection of circulating S100 proteins can be used in methods for diagnostic and prognostic evaluation of breast cancer subjects. Additionally, increased levels of circulating autoantibodies reactive against S100 proteins were detected in the serum of cancer subjects.

### 5.1. ASSAYS FOR DETECTION OF S100 EXPRESSION

Measurement of levels of S100 proteins in Serum or body fluids can be used for the early diagnosis of diseases such as cancer. Moreover, the monitoring of S100 protein levels can be used prognostically to stage the progression of the disease and to evaluate the efficacy of compounds used to treat a cancer subject.

The detection of S100 proteins in a body fluid from a subject can be accomplished by any of a number of methods. Preferred diagnostic methods for the detection of S100 proteins in the serum of a patient can involve, for example, immunoassays wherein S100 proteins are detected by their interaction with an S100 specific antibody. Antibodies useful in the present invention can be used to quantitatively or qualitatively detect the presence of S100 peptides. In addition, reagents other than antibodies, such as, for example, polypeptides that bind specifically to S100 proteins can be used in assays to detect the level of S100 protein expression.

Immunoassays to be used include but are not limited to assay systems using techniques such as Western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement fixation assays, immunoradiometric assays, fluorescent immunoassays, protein A immunoassays, to name but a few.

A biological sample which may contain S100 proteins, such as serum or other biological fluids in which secreted proteins can localize, is obtained from a subject suspected of having a particular cancer or risk for cancer. Immunoassays for detecting expression of S100 protein typically comprise contacting the biological sample, such as a serum sample derived from a subject, with an anti-S100 antibody under conditions such that specific antigen-antibody binding can occur, and detecting or measuring the amount of any immunospecific binding by the antibody. In a specific aspect, such binding of antibody, for example, can be used to detect the presence and increased expression of S100 proteins wherein The detection of increased expression of S100 proteins is an indication of a diseased condition. The levels of S100 protein in a serum sample are compared to norms established for normal individuals and for subjects with a variety of non-cancerous or pre-cancerous disease states.

The biological sample, such as a serum sample is brought in contact with a solid phase support or carrier, such as nitrocellulose, for the purpose of immobilizing any proteins present in the sample. The support is then washed with suitable buffers followed by treatment with detectably labeled S100 specific antibody. The solid phase support is then washed with the buffer a second time to remove unbound antibody. The amount of bound antibody on the solid support is then determined according to well known methods. Those skilled in the art will be able to determine optional assay conditions for each determination by employing routine experimentation.

One of the ways in which S100 antibodies can be detectably labeled is by linking the antibody to an enzyme, such as for use in an enzyme immunoassay (E1A) (Voller, A., "The Enzyme Linked Immunosorbent Assay (ELISA)", 1978, Diagnostic Horizons 2:1-7, Microbiological Associates Quarterly Publication, Walkersville, MD; Voller, A., et al., 1978, J. Clin. Pathol. 31:507-520; Butler, J.E., 1981, Meth. Enzymol. 73:482-523). The enzyme which is bound to the antibody will react with an appropriate substrate, preferably a chromogenic substrate, in such a manner as to produce a chemical moiety that can be detected, for example, by spectrophotometric, fluorimetric, or by visual means. Enzymes that can be used to detectable label the antibody include but are not limited to, horseradish peroxidase and alkaline phosphatase. The detection can also be accomplished by colorimetric methods that employ a chromogenic substrate for the enzyme.

Detection of S100 antibodies may also be accomplished using a variety of other methods. For example, by radioactively labeling the antibodies or antibody fragments, it is possible to detect S100 protein expression through the use of a radioimmunoassay (RIA) (see, for example, Weintraub, B., Principles of Radioimmunoassays, Seventh Training Course on Radioligand Assay Techniques, The Endocrine Society. March 1986). The radioactive isotope can be detected by such means as the use of a gamma counter or a scintillation counter or by autoradiography.

The antibody may also be labeled with a fluorescent compound. Among the most commonly used fluorescent labeling compounds are fluorescein isothiocyanate, rhodamine, phycoerythrin and fluorescamine. Likewise, a bioluminescent compound may be used to label the S100 antibody. The presence of a bioluminescence protein is determined by detecting the presence of luminescence. Important bioluminescence compounds for purposes of labeling are luciferin, luciferase and aequorin.

Expression levels of S100 proteins in biological samples can be analyzed by two-dimensional gel electrophoresis. Methods of two-dimensional electrophoresis are known to those skilled in the art. Biological samples, such as serum samples, are loaded onto electrophoretic gels for isoelectric focusing separation in the first dimension which separates proteins based on charge. A number of first-dimension gel preparations may be utilized including tube gels for carrier amphotytes-based separations or gels strips for immobilized gradients based separations**.** After first-dimension separation, proteins are transferred onto the second dimension gel, following an equilibration procedure and separated using SDS PAGE which separates the proteins based on molecular weight. When comparing serum samples derived from different subjects, multiple gels are prepared from individual serum samples.

Following separation, the proteins are transferred from the two-dimensional gels onto membranes commonly used for Western blotting. The techniques of Western blotting and subsequent visualization of proteins are also well known in the art (Sambrook et al, "Molecular Cloning, A Laboratory Manual", 2nd Edition, Volume 3, 1989, Cold Spring Harbor). The standard procedures may be used, or the procedures may be modified as known in the art for identification of proteins of particular types, such as highly basin or acidic, or lipid soluble, etc. (See for example, Ausubel, et al.,1989, Current Protocols in Molecular Biology, Green Publishing Associates and Wiley Interscience. N.Y.). Antibodies that bind to the S100 proteins are utilized in an incubation step, as in the procedure of Western blot analysis. A second antibody specific for the first antibody is utilized in the procedure of Western blot analysis to visualize proteins that reacted with the first antibody.

The detection of S100 protein expression can also be used to monitor the efficacy of potential anti-cancer compounds during treatment. For example, the level of S 100 protein expression can be determined before and during treatment. The efficacy of the compound can be followed by comparing S100 expression throughout the treatment. Compounds exhibiting efficacy are those which decrease the level of S100 protein expression as treatment with the compound progresses.

Elevated levels of an S100 protein was detected in serum samples derived from cancer subjects. In particular, increased levels of S100-A9 were detected in serum samples derived from colon and lung patients. In addition. S100-A7 and S100-A8 proteins were shown to be secreted by breast cancer cells, which provide the basis for diagnostic and prognostic assays for breast cancer. The detection and/or quantitative measurement of S100 proteins in serum or other body fluids can be used in screening of subjects who are at risk for developing certain types of cancers or other proliferative disorders in which the S100 proteins are over expressed. In addition qualitative differences in the pattern of occurrence in serum or biological fluids of different members of the S100 family of proteins can be used as a screening diagnostic or prognostic indicator of cancer or cancer risk.

### 5.2. ASSAYS FOR DETECTION OF ANTI-S 100 AUTOANTIBODIES

The present invention provides diagnostic and prognostic methods for diseases such as cancer based on detection of circulating S100 autoantibodies in a subject. The method is validated by the use of a biological sample from a subject with cancer and from controls without cancer. A biological sample which may contain autoantibodies. Such as serum, is obtained from a subject suspected of having a particular cancer or suspected of being predisposed to developing cancer. A similar body fluid is obtained from a control subject that does not have cancer.

In accordance with the invention, measurement of autoantibodies reactive against the S100 protein antigens can be used for the early diagnosis of diseases such as cancer. Moreover, the monitoring of autoantibody levels can be used prognostically to stage the progression of the disease. The detection of autoantibodies in a serum sample from a patient can be accomplished by any of a number of methods. Such methods include immunoassays which include, but are not limited to, assay systems using techniques such as Western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement fixation assays, immunoradiometric assays, fluorescent immunoassays, protein A immunoassays, to name but a few.

Such an immunoassay is carried out by a method comprising contacting a serum sample derived from a subject with a sample containing the S100 protein antigens under conditions such that specific antigen-antibody binding can occur, and detecting or measuring the amount of any immunospecific binding by the autoantibody. In a specific aspect, such binding of autoantibody by tissue sections, for example, can be used to detect the presence of autoantibody wherein the detection of autoantibody is an indication of a diseased condition. The levels of autoantibodies in a serum sample are compared to the levels present in an analogous serum sample from a subject not having the disorder.

The immunoassays can be conducted in a variety of ways. For example, one method to conduct such assays involves anchoring of S100 protein onto a solid support and detecting anti-S100 antibodies specifically bound thereto. The S100 proteins to be utilized in the assays of the invention can be prepared via recombinant DNA techniques well known in the art. For example, in instances where the nucleotide sequence of a DNA encoding an S100 protein is available, the DNA can be genetically engineered into an appropriate expression vector for large scale preparation of S100 protein. It may be advantageous to engineer fusion proteins that can facilitate labeling, immobilization or detection of the S 100 protein. See, for example, the techniques described in Sambrook et al., 1989, Molecular Cloning: A laboratory Manual, Cold Spring Harbor Press, Cold Spring Harbor, N. Y. Alternatively the S100 protein may be purified from natural sources, e.g., Purified from cells, using protein separation techniques well known in the art. Such purification techniques may include, but are not limited to molecular sieve chromatography and/or ion exchange chromatography. In practice, microtitre plates are conveniently utilized as The solid support for the S100 proteins. The surfaces may be prepared in advance and stored.

The present invention is demonstrated by way of example wherein elevated levels of circulating autoantibodies reactive against several S 100 protein antigens were detected in the sera of cancer patients. The detection and/or quantitative measurement of circulating anti-S100 autoantibodies in serum can be used in screening of subjects who are at risk for cancer or other proliferative disorders in which S100 proteins are expressed. The identified protein antigens can be used as immunogens for stimulation of an host immune response against the tumor cells. It is expected that such an approach can be used as a method for inhibiting tumor cell growth or facilitating tumor cell killing in individuals with specific cancers.

### 5.3. IMMUNOTHERAPY

S100 proteins can be used as antigens to immunize patients suffering from diseases characterized by the expression of the S100 protein antigens. Stimulation of an immunological response to such antigens, is intended to elicit a more effective attack of tumor cells; such as inter alia inhibiting tumor cell growth or facilitating the killing of tumor cells. The identification of autoantibodies to S100 protein antigens associate with particular cancers provides a basis for immunotherapy of the disease.

The patient may be immunized with the S100 protein antigens to elicit an immune response which facilitates killing of tumor cells or inhibiting tumour cell growth. The S100 protein antigens can be prepared using the methods described above for purification of proteins.

An immunogen comprising a purified S100 protein antigen to which a patient with cancer has developed autoantibodies, is used to elicit an immune response. For administration, the S100 protein antigens should be formulated with a suitable adjuvant in order to enhance the immunological response to the protein antigen Suitable adjuvants include, but are not limited to mineral gels, e.g. aluminum hydroxide, surface active substances such as lysolecithin, pluronic polyols, polyanions, peptides, oil emulsions, and potentially useful human adjuvants such as BCG (*bacilli Calmeu-Guerin*) and (*Corynebacterium parvum*). Many methods may be used to introduce the formulations derived above; including but not limited to oral, intradermal, intramuscular, intraperitoneal, intravenous, and subutaneous.

### 5.4. KITS

The assays described herein can be performed, for example, by utilizing pre-packaged diagnostic kits, comprising at least an S100 peptide (for detection of S100 autoantibodies) or an S100 antibody reagent (for detection of S100 protein), which can be conveniently used, *e.g*., in clinical settings to diagnose disorders such as cancer.

A kit may comprise components for detecting and/or measuring human IgG antibodies directed toward S 100 antigen. As one example where the antibodies are detected and/or measured by enzyme linked immunoabsorbent assay (ELISA), such components may comprise target antigen, in the form of at least one and preferably a plurality of different S100 antigens or epitopes thereof, linked to a solid phase, and a means for detecting a human antibody antibody bound to target antigen. Such means for detection may be for example, an antibody directed toward the constant region of human IgG (*e.g.,* rabbit anti-human IgG antibody), which may itself be detectably labeled (*e.g.,* with a radioactive; fluorescent, colorimetric or enzyme label), or which may be detected by a labeled secondary antibody (*e.g..* goal anti-rabbit antibody).

A kit may comprise components which detect and/or measure S100 antigens in the biological sample of a subject. For example, where S100 proteins are detected and/or measured by enzyme linked immunoabsorbent assay (ELISA), such components may comprise an antibody directed to epitopes of the S 100 proteins which can be used to detect and/or quantitate the level of S 100 expression in the biological sample. The antibody itself may be detectably labeled with a radioactive, flourescent, colorimetric or enzyme label. Alternatively, the kit may contain a labeled secondary antibody.

### 6. EXAMPLE: DETECTION OF A TUMOR ANTIGEN USING SERUM ISOLATED FROM A SUBJECT HAVING CANCER

Subjects with lung and colon cancer were assayed for detection of elevated levels of S 100 protein. Tumor tissue as well as normal tissue control was obtained from subjects with the following types of cancer: small cell lung cancer, squamous cell carcinoma of the lung, adenocarcinoma of the lung, esophageal cancer and Barrett's metaplasia, colon cancer, pancreatic cancer, breast cancer, and prostate cancer. An aliquot of whole tissue was solubilized using a solubilization cocktail prior to two-dimensional electrophoresis.

Solubilized proteins were loaded onto electrophoretic gels for separation in the first dimension, based on charge. Two different first dimension gel preparations were utilized namely tube gels for carrier ampholyte based isoelectric focusing (IEF) and gels strips for immobilized pH gradients based separations (IPG). After the first dimension separation, proteins were transferred onto the second dimension gel, following an equilibration procedure and separated in the second dimension based on molecular weight. In some cases multiple gels were prepared from individual serum samples. Proteins patterns were visualized by silver staining and analyzed for differences between normal and tumor tissue and/or for differences between different tumor types by means of a computerized matching process and by means of quantitation of spot intensities.

Many proteins occurred at an increased intensity in particular tumors compared to controls. A set of four proteins occurred at an increased intensity in tumor patterns which were not detectable in controls. The quantitative data in lung cancer for one of the proteins which was later identified as S 100-A8 is given in Table I.

**TABLE I**

| Detection of S100-A8 in Tissue From Lung Tumor Patients | | | |
|---|---|---|---|
| Patient | Type | Number | Integrated Intensity (Mean) |
| Tumor | | | |
| | Squamous | 9 | 1.84 |
| | Adenocarcinoma | 8 | 1.31 |
| | Small Cell | 9 | 0.34 |
| Normal Tissue | | 26 | Not Detected |

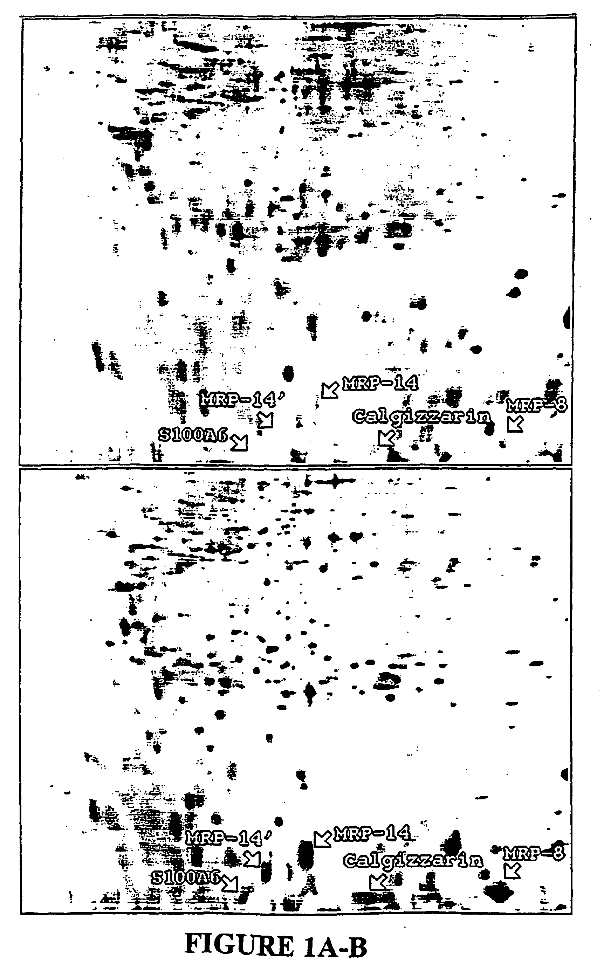

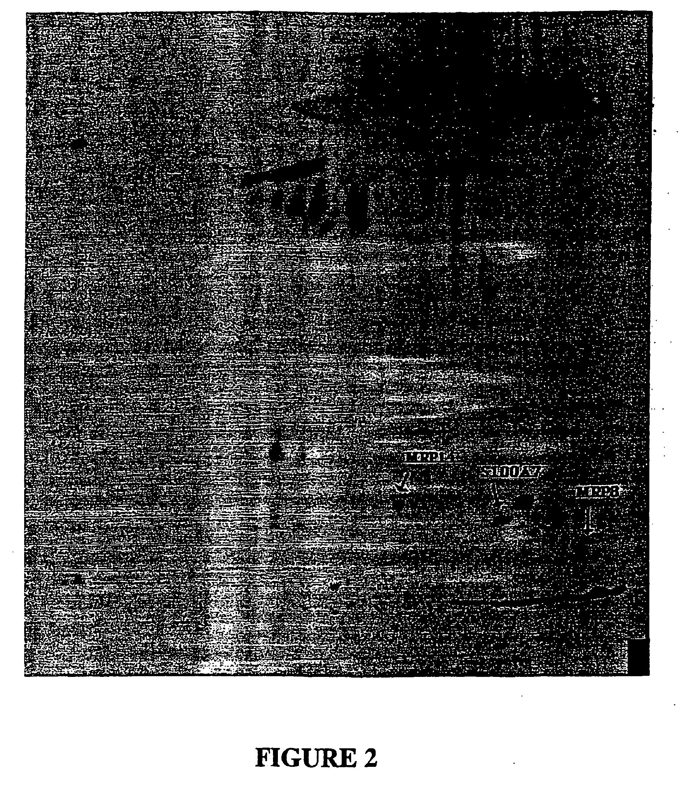

This protein was extracted from 2-D gels and subjected to amino acid sequencing. It gave the N-terminal amino acid sequence MILTELEKALN, which is 100% identical with the reported N-terminal amino acid sequence of the S 100-A8 protein, found by searching protein databases. The other three proteins in the set were also identified as belonging to the S100 family of proteins. Two were consistent, with S 100-A9 which has two translation initiation sites situated 4 amino acids apart, and one with Calgizzarin which is another S100 related protein, as determined by reactivity with monoclonal antibodies and by comparison with published figures of two-dimensional protein separations. The location of these proteins in 2-D patterns of different tumor types is provided in Figures 1 and 2.

A commercially available antibody against S 100-A9 (DAKO;Carpinteria, CA) was utilized for immunohistochemistry and for Western blotting. Immunohistochemistry on sections of tumor tissue and corresponding normal tissue from the same subject with lung cancer was done. The analyzed tissue sections revealed minimal S100-A9 immunoreactivity in the normal lung tissue. The observed increase in reactivity in the tumor tissue was attributed to the presence of infiltrative cells. There was also marked immunoreactivity in the area of normal tissue immediately adjacent to the tumor, resulting from infiltrative cells (i.e., ganulocytes, monocytes and/or macrophage) recruited to the tumor.

An assay was performed to determine whether the S100-A9 antibody would recognize a specific band in the serum of tumor subjects, at levels greater than that which might be present in the serum of normal individuals. Proteins contained in the serum of 14 lung tumor subjects and 14 normal individuals were separated by one dimensional gel electrophoresis, the proteins transferred to PVDF membranes and probed with a commercial antibody (DAKO;Carpinteria, CA) that is reactive against the S100-A9 protein. Integrated intensity analysis of reactivity in a band visualized at 14 kDa revealed markedly increased reactivity in the serum from tumor subjects (n=14; mean intensity of 0.46) as compared to that in the serum from normal individuals (n=14; mean intensity of 0.09) (Table II). Another band at 22 kDa was also observed in some tumors and was presumed to result from another immunoreactive form of S 100 protein. Similar results were also observed with serum of subjects with colon cancer.

**TABLE II**

| Analysis of MRP14 Detection in Serum From Lung Tumor Patients | | | | |
|---|---|---|---|---|
| Patient | Type | Stage | Number | Integrated Intensity (Mean) |
| Tumor | All | | 14 | 0.46 |
| | Squamous | I | 4 | 0.65 |
| | Adenocarcinoma | None | 1 | 0.03 |
| | | I | 3 | 0.26 |
| | | IIIA | 2 | 0.42 |
| | Bronchoalveolar | I | 1 | 0.44 |
| | Small Cell | None | 2 | 0.65 |
| | Other (Met. Ovarian) | None | 1 | 0.01 |
| Normal | | | 14 | 0.09 |

A major determinant of the potential of a protein synthesized by tumors to be detected in serum and other biological fluids is its secreted nature. The features of a protein that determine whether it is secreted by cells remains poorly understood. Factors that affect secretory process may depend on the occurrence of post-translational modifications in the protein as well as the activation of certain signaling pathways. In order to facilitate the identification of secretory proteins, a method has been developed to purify proteins secreted by cultured tumor cells, visualize the proteins in two-dimensional gels by coomassie or silver staining and analyze secreted protein spots by N-terminal sequencing or by Mass Spectrometry of their constituent peptides. Using this process three members of the S 100 family were identified as secreted proteins in breast cancer. N-terminal sequencing of two secreted proteins identified them as MRP8 and MRP 14 based on the following sequences:

| | |
|---|---|
| Amino Acid Sequence | Identify |
| MLTELEKALN | MRP8 |
| MCKMSQECRN | MRP14 |

Additionally, mass spectrometry identified S100A7 as a secreted protein in breast cancer. Seven peptides masses obtained by mass spectrometry matched expected peptide masses for S100A7/These were: 1291.65; 1307.66; 1323.65; 1384.69; 1455.66; 1583.65; and 1711.91.

### 7. EXAMPLE: DETECTION OF AUTOANTIBODIES SPECIFIC FOR S100 PROTEINS IN THE SERA OF SUBJECTS WITH CANCER

Using the method of the present invention, sera from subjects with lung cancer were screened for reactivity against S100 proteins.

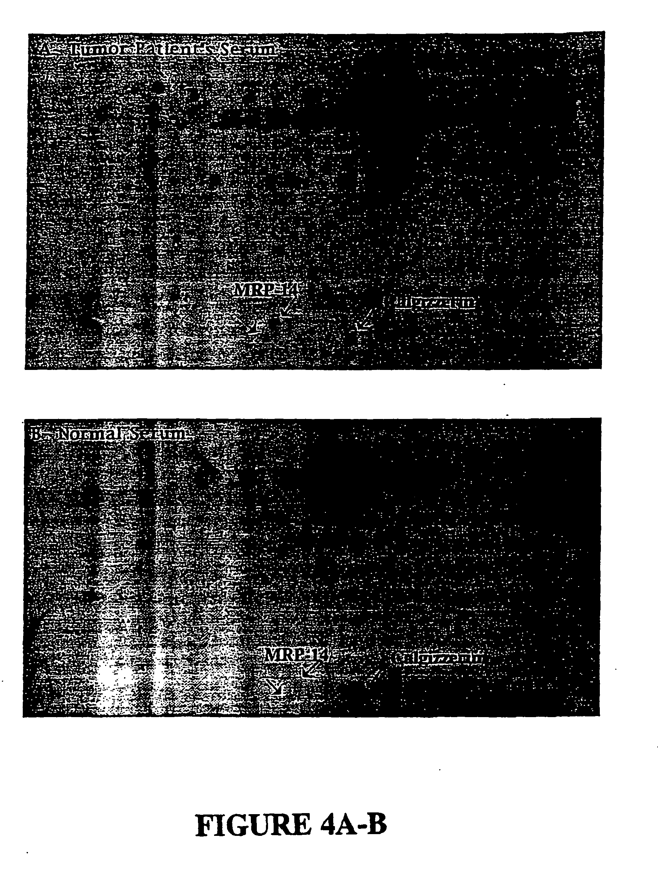

An aliquot of lung tumor tissue was solubilized in a urea cocktail (per liter:8M area, 20 ml Nonidet P-40 surfectant, 20 ml of ampholytes (pH 3.5-10), 20ml of 2-mercuptoethanol and 0.2mm of phenylmethylsulfonyl fluoride (PMSF) in distilled demonize H₂O) and 40 micrograms of solubilized protein was loaded onto a carrier ampholyte based (pH 3.8) tube gel and separated in the first dimension for 12,000 volt hours. Following an equilibrium step, the first dimension tube gel was loaded onto a cassette containing the second dimension gel. Electrophoresis in the second dimension was complete when the tracking dye present in the equilibration buffer reached the opposite end of the second dimension gel, in relation to the first dimension gel. Following electrophoresis the separated proteins were transferred onto a polyvinylideme fluoride (PVDF) membrane (millipore). The membrane was preincubated with a blocking buffer and subsequently incubated with serum obtained from a subject with lung adenocarcinoma, which was diluted 1:100 in the buffer solution (Tris-buffered-saline containing .01% Between 20 and 1.8 gm/100 ml non-fat dry milk), for 1 hr at room temperature. After three washes with a buffer solution, the membrane was incubated for 1 hr with a horseradish peroxidase conjugated rabbit anti-human antibody (available from Amersham). Reactive proteins were revealed by a chemiluminescent technique. The sera sample from the cancer subject was found to be reactive against a set of S100 proteins identified by microsequencing as S100-A9 and calgizzarin (Figure 4A-B).

## Claims

1. A method for diagnosis of a subject with lung cancer comprising:
(a) contacting a biological fluid sample derived from a subject with a sample containing S100 protein antigens selected from the group consisting of S100-A9 and calgizzarin under conditions such that a specific antigen-antibody binding can occur, and
(b) detecting immunospecific binding of the autoantibodies to said protein in the subject's biological fluid sample, wherein the presence of autoantibodies indicates the presence of lung cancer.

2. Use of an S100 protein selected from the group consisting of, S100-A9 and calgizzarin for the detection of elevated levels of autoantibodies to the protein in a biological fluid sample from a subject with lung cancer.

## Patentansprüche

1. Diagnoseverfahren für einen Patienten mit Lungenkrebs, das umfasst:
(a) Herstellen eines Kontakts zwischen einer Probe eines biologischen Fluids, die von einem Patienten gewonnen worden ist, und einer Probe, die S100-Protein-Antigene enthält, die aus der Gruppe ausgewählt sind, die aus S100-A9 und Calgizzarin besteht, unter Bedingungen, derart, dass eine spezifische Antigen-Antiktörper-Bindung auftreten kann; und
(b) Detektieren einer immunospezifischen Bindung der AutoAntikörper mit dem Protein in der Probe des biologischen Fluids des Patienten, wobei das Vorhandensein von Auto-Antikörpern das Vorhandensein von Lungenkrebs anzeigt.

2. Verwendung eines S100-Proteins, das aus der Gruppe ausgewählt ist, die aus S100-A9 und Calgizzarin besteht, für die Detektion eines erhöhten Pegels von Auto-Antikörpern relativ zu dem Protein in einer Probe eines biologischen Fluids von einem Patienten mit Lungenkrebs.

## Revendications

1. Procédé pour le diagnostic d'un sujet atteint d'un cancer du poumon, comprenant .
(a) La mise en contact d'un échantillon de fluide biologique provenant d'un sujet avec un échantillon contenant des antigènes de protéine S100 sélectionnés à partir du groupe constitué de S100-A9 et de calgizzarine dans des conditions telles qu'une liaison spécifique antigène-anticorps puisse se produire ; et
(b) La détection d'une liaison immunospécifique des auto-anticorps à ladite protéine dans l'échantillon de fluide biologique du sujet, dans lequel la présence d'auto-anticorps indique la présence d'un cancer du poumon.

2. Utilisation d'une protéine S100 sélectionnée à partir du groupe constitué de S100-A9 et de calgizzarine en vue de la détection de niveaux élevés d'auto-anticorps à la protéine dans un échantillon de fluide biologique provenant d'un sujet atteint d'un cancer du poumon.
